# EUROPÄISCHE PATENTSCHRIFT

(11) **EP 0 496 089 B1**
(45) Veröffentlichungstag und Bekanntmachung des Hinweises auf die Patenterteilung: **26.05.1993**
(21) Anmeldenummer: 91121905.3
(22) Anmeldetag: 01.01.1992
(51) Int. Cl.: A61F 2/36, A61F 2/30

(54) **Hüftkopf-Schaft-Prothese**
Stem for a hip joint prosthesis
Tige pour prothèse de la hanche

(30) Priorität: 21.01.1991 DE 4101587
(43) Veröffentlichungstag der Anmeldung: 29.07.1992
(73) Patentinhaber: Baumann, Friedrich, D-86633 Neuburg (DE)
(72) Erfinder: Baumann, Friedrich, D-86633 Neuburg (DE)
(74) Vertreter: Neubauer, Hans-Jürgen, Dipl.-Phys.

(56) Entgegenhaltungen:
- EP-A- 0 159 510
- EP-A- 0 315 283
- EP-A- 0 357 546
- EP-A- 0 359 097
- DE-A- 3 247 726
- US-A- 4 156 943

## Beschreibung

Die Erfindung betrifft eine Hüftkopf-Schaft-Prothese gemäß dem Oberbegriff des Anspruchs 1, wie sie aus der DE-A-3 415 934 bekannt ist. Insbesondere betrifft die Erfindung eine Hüftkopf-Schaft-Prothese mit einem länglichen Schaftteil, das im Markraum eines Oberschenkelhalsstumpfes implantierbar und durch eine das Schaftteil umgebende Zementschicht im Markraum fixierbar ist.

Eine Hüftkopf-Schaft-Prothese wirkt in an sich bekannter Weise mit einer in den Beckenknochen einschraubbaren Pfanne zusammen, die ein Außengewinde an einer Titan-Außenschale aufweist. Die Außenschale ist bevorzugt konisch geformt, um einen zusätzlich zum Gewindekontakt erhöhten Halt zu erreichen. Die Innenschale der Pfanne ist aus Polyäthylen, das vor allem bei Paarung mit einer Keramikausführung des Hüftkopfs die besten tribologischen Werte ergibt.

Probleme ergaben sich bisher insbesondere hinsichtlich der primären Stabilität und Langzeitstabilität des Schaftteils im Oberschenkelmarkraum, wobei grundsätzlich zwei Arten der Fixierung bekannt sind:

In einem ersten Verfahren, das früher regelmäßig durchgeführt wurde, wird das Schaftteil mit Hilfe einer Zementverbindung im Oberschenkelmarkraum fixiert. Die Erfindung betrifft eine Hüftkopf-Schaft-Prothese für diese Art der Fixierung, die auch heute dann angewendet wird, wenn eine frühzeitige Belastung des Hüftgelenks, z.B. bei älteren Personen, erforderlich ist.

Das zweite Verfahren besteht in der zementfreien Implantation einer selbsteinheilenden Hüftkopf-Schaft-Prothese. Dazu ist es bekannt, die Oberfläche mit Hydroxyl-Apatit zu beschichten, das die Knochenneubildung induziert und die Einheilung des Prothesenschaftes fördert. Schwierigkeiten ergeben sich dadurch, daß der Markraum des Oberschenkelhalses zur Aufnahme des Prothesenschaftes bei jeder Person physiologisch unterschiedlich, teilweise nach vorne und außen gebogen und auch in seinem Querschnitt unterschiedlich geformt ist. Daher sind bekannte Prothesenschäfte mit einer Reihe von unterschiedlichen Formen ausgeführt worden, insbesondere wurden elliptische Querschnitte und sogenannte physiologische Krümmungen realisiert.

Allen diesen Ausführungen für eine zementfreie Implantation ist gemeinsam, daß die Prothesenschäfte nur punktuell oder mit sehr kleinflächigem Kontakt im Markraum des Oberschenkelhalses anliegen und die Einheilung durch den vorerst nur wenig stabilen Sitz des Prothesenschaftes verzögert und behindert wird.

Um hier eine Verbesserung zu schaffen, wurde für eine zementfreie Implantation bereits eine Hüftkopf-Schaft-Prothese bekannt (EP-A-359 097), bei der das Schaftunterteil zylindrisch geformt und in einem im gleichen Durchmesser zylindrisch aufgebohrten Markraum des Oberschenkelhalsstumpfes implantierbar ist. Dadurch werden große Kontaktflächen mit großer, primärer Stabilität und verbessertem, knöchernen Einbau erreicht.

Die Hauptprobleme bei einer einzementierbaren Ausführung einer Hüftkopf-Schaft-Prothese sind dagegen im wesentlichen anders gelagert. Für eine Fixierung des Prothesenschafts wird dieser mit seinen relativ glatten Oberflächen in den mit Zement vorbereiteten Markraum eingeschoben. Eine bekannte Ausführungsform (DE-A-32 47 726) weist einen sich nach unten konisch verjüngenden Schaft auf. Der Schaft stützt sich dabei mit Teilen seiner Umfangsfläche direkt an der Innenwand des Markraums ab, so daß dort eine direkte Anlage ohne eine dazwischenliegende Zementschicht erfolgt. Durch die fehlende Zementschicht ist im Bereich der direkten Anlage keine Anbindung gegeben, so daß dadurch die erforderliche, feste Halterung reduziert wird. Ein wesentlicher Nachteil besteht aber insbesondere auch dadurch, daß sich der Spalt für die Zementschicht anschließend an die direkten Anlagestellen erst ganz allmählich verbreitert, so daß dort die Zementschicht sehr dünn ist und in ihrer Schichtdicke erst in einem weiteren Abstand von der Anlagestelle zunimmt. Diese sehr dünne Zementschicht im unmittelbaren Bereich der direkten Anlagestellen hat wegen ihrer zu geringen, inneren Stabilität die Tendenz, sich abzulösen und zu zerbröseln. Dadurch werden im angrenzenden Bereich an die Anlagestellen die Verbindung und der feste Halt weiter reduziert. Zudem wirken solche abgelösten Zementteilchen toxisch und zerstörend auf den umgebenden Knochen.

Um eine Verbesserung hinsichtlich gleichmäßiger Zementschichtdicken zu erreichen, ist es bereits bekannt, in den Markraum einen Kunststoffstern bis zur Lage der Unterseite des Prothesenschaftes einzuschieben und in diesem Kunststoffstern den Unterteil des Prothesenschaftes im Markraum zu zentrieren. Dazu ist der Prothesenschaft durchbohrt und wird über eine mit dem Kunststoffstern verbundene Führungsstange, die durch diese Bohrung gesteckt ist, in den Markraum eingesetzt. Die Führungsstange wird nach der Implantation wieder herausgenommen. Dieses Verfahren ist ersichtlich kompliziert und aufwendig, wobei trotz des Kunststoffsterns nicht sichergestellt werden kann, daß der Unterteil des Prothesenschafts in der Aufnahme im Kunststoffstern während der Aushärtezeit des Zements gut festsitzt und zentriert ist. Zudem bleibt der Kunststoffstern in nachteiliger Weise als weiteres, körperfremdes Teil ständig im Oberschenkelhalsstumpf.

Weiter sind Riffelungen und Profilierungen von Schaftoberflächen bekannt:

Für eine zementfreie Implantation sind kleinflächige, relativ eng aneinanderliegende Erhebungen an der gesamten Schaftoberfläche bekannt, die eine Verbesserung bei der Verankerung des Prothesenschafts bewirken sollen (CH-A-675 826; DE-A-32 16 539; DE-A-34 15 934 und DE-U-81 24 912). Eine solche Oberflächenstruktur mit einer Vielzahl relativ eng aneinanderliegender Erhebungen kann Vorteile für die Verankerung bei einer zementfreien Implantation erbringen; bei einer Einzementierung des Prothesenschafts ist eine solche Oberflächenstruktur nicht geeignet, da der Zementschichtbereich mit einer Vielzahl von Verdünnungen und Übergängen immer wieder unterbrochen wäre und die vorstehend in diesem Zusammenhang geschilderten Nachteile an einer Vielzahl von Stellen auftreten würden.

Weiter ist eine Strukturierung der Schaftoberfläche mit Längsnuten bekannt (DE-U-88 11 758), die eine Verbesserung der Zementverzahnung zwischen Markraum und Schaftoberfläche erreichen sollen. Diese Längsnuten sind aber relativ tief eingeschnitten, so daß an den Übergangsstellen zwischen Nuten und Umfangsflächen große Sprünge in der Zementschichtdicke entstehen, die die Stabilität negativ beeinflußen.

Aufgabe der Erfindung ist es, eine gattungsgemäße, einzementierbare Hüftkopf-Schaft-Prothese so weiterzubilden, daß eine verbesserte primäre und Langzeitstabilität erreichbar ist.

Diese Aufgabe wird mit den kennzeichnenden Merkmalen des Anspruchs 1 gelöst.

Gemäß Anspruch 1 ist das Schaftunterteil zylindrisch geformt. Im unteren Bereich des Schaftunterteils sind am Umfang verteilt Erhebungen als untere Abstandshalter angebracht. Diese zentrieren den unteren Bereich des Schaftunterteils in einem Markraum des Oberschenkelhalsstumpfes als Aufnahmeraum, der zylindrisch im Durchmesser des Außendurchmessers des Schaftunterteils zuzüglich der abstehenden Abstandshalter aufgebohrt ist. Das Schaftoberteil ist an einer Seite als gerade Verlängerung des zylindrischen Schaftunterteils ausgebildet. Im Bereich des Schaftoberteils ist die Medialseite konisch mit einem konkaven Bogen erweitert. Dort ist wenigstens eine Erhebung als oberer Abstandshalter zur Anlage an die Corticalis im Markraum angebracht.

Aufgrund dieser Abstandshalteranordnung wird ein gleichmäßiger Spalt und damit eine gleichmäßige Zementschichtdicke mit nur geringen Unterbrechungen an den wenigen Abstandshaltern zwischen Aufnahmeraum und Schaft für eine hohe primäre und Langzeitstabilität geschaffen.

Der Zement bricht unter bestimmten Schichtdicken und bei Schichtdifferenzbereichen. Bei Zementbruch ergeben sich im Bruchbereich die bekannten Knochenschäden wegen der Toxizität der Bruchstücke und eine Knochenauflösung, was bisher ein maßgeblicher Grund für häufige Schaftlockerungen war.

Diesem Problem wird erfindungsgemäß dadurch begegnet, daß über die ganze Schaftlänge eine gleichmäßige Zementdicke geschaffen wird. Durch die zylindrische Schaftausbildung in Verbindung mit einer zylindrischen Aufbereitung des Markraums ist es ausreichend, wenn nur wenige Abstandshalter für eine Zentrierung am unteren Schaftende vorgesehen werden. Im oberen Bereich ist für die Zentrierung ein Abstandshalter zur Anlage an der Corticalis medial des Schenkelhalsstumpfes ausreichend.

Wegen der wenigen, kleinen Abstandshalter in großer Distanz ist die Zementschicht nur an entsprechend wenigen Stellen und insbesondere nicht in der ganzen Ausdehnung der Länge und des Umfangs unterbrochen.

In einer bevorzugten Ausführungsform nach Anspruch 2 sind die Abstandshalter in der Form von in Längsrichtung des Schaftteils verlaufenden, schmalen, im Vergleich zur Schaftlänge jedoch relativ kurzen Noppenreihen ausgebildet. Zwischen den Noppen sind Zwischenräume vorgesehen. Um mögliche Durchmessertoleranzen nach der zylindrischen Vorbereitung des Aufnahmeraums auszugleichen und für eine sichere Zentrierung bezüglich der Zylindermittenachse sind Noppenreihen mit einer gewissen Längserstreckung verwendet. Um durch diese Längserstreckung der Noppenreihen die Zementschicht möglichst wenig zu unterbrechen, sind zwischen den Noppen Zwischenräume für einen Durchgang der Zementschicht vorgesehen.

Nach Anspruch 3 sind drei am Umfang des Schaftunterteils verteilte, untere Abstandshalter bzw. Noppenreihen vorgesehen. Für eine Zentrierung sind drei solche Abstandshalter ausreichend, die dann vorteilhaft weit in einem Winkelabstand von 120° auseinander liegen und die Zementschicht in weiten Umfangsbereichen nicht unterbrechen.

Gemäß Anspruch 4 sind die Abstandshalter bzw. die Noppenreihen gegeneinander in der Schaftlängsrichtung bzw. in ihrer Höhe versetzt angebracht. Dadurch wird vorteilhaft eine möglichst geringe Umfangsunterbrechung der Zementschicht aufgrund des Höhenversatzes erreicht.

Die Höhe der Abstandshalter bzw. der Noppen ist nach Anspruch 5 zwischen einer erforderlichen Mindestdicke der Zementschicht von ca. 1,5 mm und ca. 3,5 mm zu wählen. Besonders gute Ergebnisse werden bei einer bevorzugten Höhe von 2,5 mm erreicht.

Zur weiteren Erhöhung der Stabilität wird nach Anspruch 6 vorgeschlagen, die Schaftoberfläche rauh mit einer bevorzugten Porentiefe von 0,2 bis 0,6 mm zu gestalten. Vorteilhaft wird auch der Markraum an seiner Innenseite mit Rillen in Form von Spiralrillen oder Längsrillen versehen. Diese Rillen können beispielsweise mit einem speziell geformten Bohrer in den Markraum eingebracht werden. Die Aufrauhung der Schaftoberfläche kann durch Sandstrahlen erreicht werden. Wegen der rauhen Struktur der Oberflächen wird eine verbesserte Zementverzahnung und damit eine erhöhte primäre und Langzeitstabilität erreicht.

In einer konkreten Ausführung nach Anspruch 7, in der gewisse Merkmale an sich bekannt sind, ist das Schaftoberteil an einer Seite als gerade Verlängerung des zylindrischen Schaftunterteils ausgebildet. Die gegenüberliegende Medialseite erweitert sich mit einem konkaven Bogen, bevorzugt mit einem Tangentenneigungswinkel von 15° gegenüber der Längsseite, so daß dadurch etwa eine Dreiecksfläche in der Dicke des Durchmessers des zylindrischen Schaftunterteils mit einer senkrechten Begrenzung, einer waagrechten Begrenzung und einer bogenförmigen Begrenzung aufgespannt ist. Das Hüftkopfteil, bevorzugt als Konus mit einer gegenüber der Längsachse um 40° geneigten Konusachse, schließt sich an der Stelle des Zusammentreffens der waagrechten und bogenförmigen Begrenzung an.

Weiter wird mit Anspruch 8 vorgeschlagen, einen an sich bekannten Kragen unterhalb des Hüftkopfteils, bevorzugt mit einer Neigung von 50° gegenüber der Längsachse, zur Auflage an der Resektionsebene des Schenkelhalses anzuformen.

Anhand eines Ausführungsbeispiels wird die Erfindung mit weiteren Einzelheiten, Merkmalen und Vorteilen näher erläutert.

Es zeigen
- Fig. 1: eine Seitenansicht einer Hüftkopf-Schaft-Prothese,
- Fig. 2: eine um 90° verdrehte Ansicht der Hüftkopf-Schaft-Prothese nach Fig. 1 und
- Fig. 3: einen Querschnitt durch eine im Markraum einzementierte Hüftkopf-Schaft-Prothese entsprechend dem Schnitt entlang der Linie A-A aus Fig. 2.

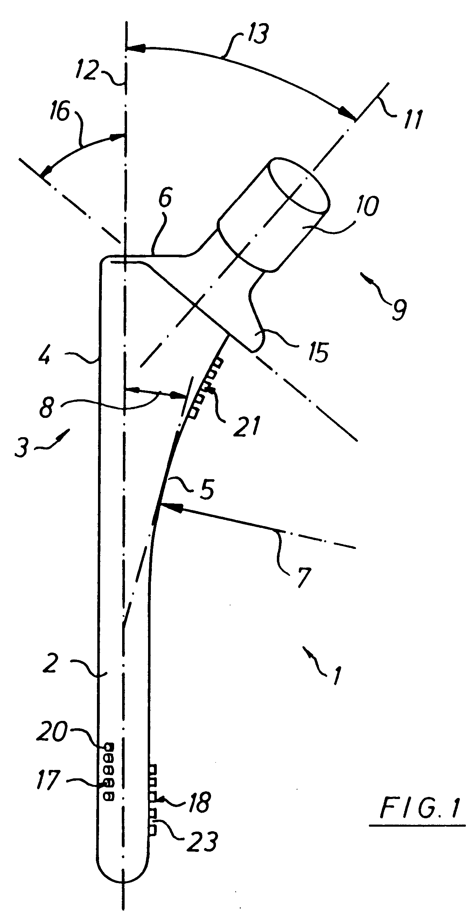

In Fig. 1 ist eine Hüftkopf-Schaft-Prothese 1 dargestellt, die aus einem unteren, zylindrisch geformten Schaftunterteil 2 und einem Schaftoberteil 3 besteht. Das Schaftoberteil 3 ist durch ein Dreieck gebildet, das durch einen, eine gerade Verlängerung des Schaftunterteils 2 bildenden, senkrechten Schenkel 4, einen konisch und konkav nach außen gebogenen Schenkel 5 und einen waagrechten, oberen Rand 6, begrenzt ist. Die Dreiecksfläche hat etwa die Dicke des zylindrischen Schaftunterteils 2.

Typische Abmessungen sind etwa eine Länge des Schaftunterteils und Schaftoberteils von 150 mm, ein zylindrischer Durchmesser des Schaftunterteils 2 von 12 mm, ein Radius 7 der konkaven Wölbung von 135 mm und ein Tangentialneigungswinkel 8 von 15°. Die Schaftlänge und der Durchmesser des zylindrischen Unterteils stehen je nach den individuellen Maßen zur Verfügung.

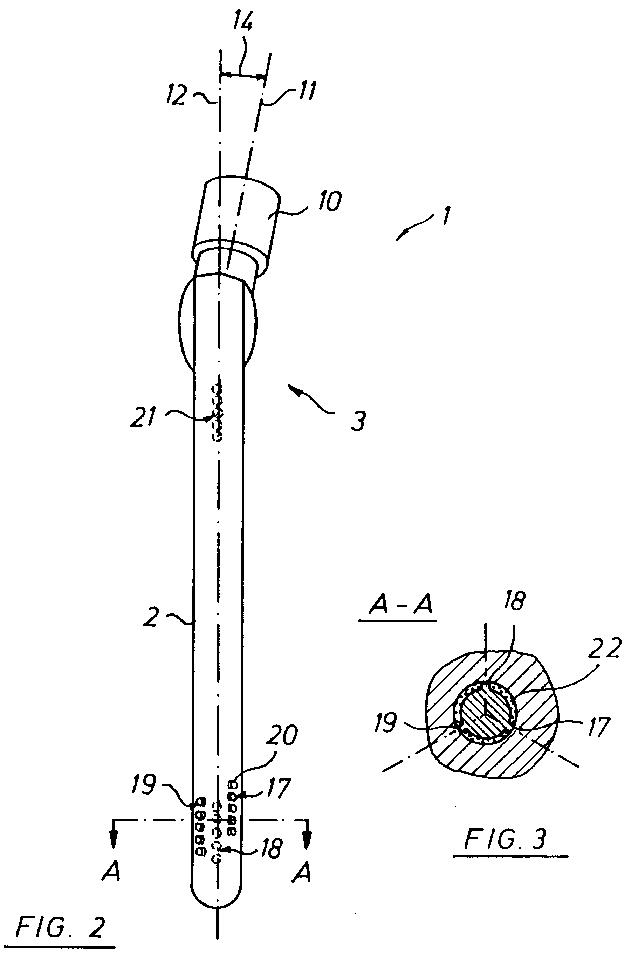

Ein Hüftkopfteil 9 besteht aus einem Konus 10, dessen Konusachse 11 mit der Längsachse 12 des Prothesenschafts einen Winkel 13 von 40° einschließt. Der Konus 10 bzw. die Konusachse 11 ist zudem um einen Winkel 14 von 10° gegenüber der Längsachse 12 bzw. der Dreiecksfläche nach vorne geneigt (sh. Fig. 2).

Unter dem Konus 10 ist ein Kragen 15 angeformt, der über den Bereich des Schaftteils seitlich übersteht und um einen Winkel 16 von 50° gegenüber der Längsachse 12 geneigt ist.

Im unteren Bereich des Schaftunterteils 2 sind am Umfang versetzt drei Längsstege als Noppenreihen 17, 18, 19 angeformt. Die Noppenreihen 17, 18, 19 bestehen jeweils aus fünf in Längsrichtung mit Zwischenräumen 23 angeordneten Noppen 20. Die Noppenreihen 17, 18, 19 sind gegeneinander in der Höhe versetzt.

Eine weitere entsprechende Noppenreihe 21 ist am konkaven Bogen 5 des Schaftoberteils 3 angeformt.

Die Noppenreihen 17, 18, 19 und 21 haben etwa eine Länge von 15 mm, die Noppen 20 eine Höhe und Breite von ca. 2,5 mm und der Zwischenraum 23 entspricht etwa der Noppenbreite.

Für die Implantation wird der Markraum des Oberschenkelknochens zur Aufnahme des Schaftunterteils 2 zylindrisch aufgebohrt, wozu an sich bekannte Werkzeuge, wie Bohrer und Fräsen, zur Verfügung stehen. Entsprechend wird der obere Bereich des Oberschenkelknochens zur Aufnahme des Schaftoberteils 3 ausgeformt. Die Größe der Aufnahme entspricht der Umfangsfläche, die durch die Außenseiten der Noppenreihen 17, 18, 19 und 21 bestimmt ist.

In den so vorbehandelten und mit Zement vorbereiteten Aufnahmeraum wird die Hüftkopf-Schaft-Prothese 1 eingesetzt, wobei sich in dem zwischen Schaftteil 2, 3 und Markrauminnenfläche gebildeten und durch die Höhe der Noppenreihen 17, 18, 19, 21 bestimmten Spalt eine Zementschicht 22 gleichmäßiger Dicke von ca. 2,5 mm ausbildet (sh. Querschnitt Fig. 3). Überschüssiger Zement wird beim Einsetzen nach oben herausgepreßt.

Die gleichmäßige Zementschichtdicke bzw. Spaltbreite wird durch nur wenige, kleinfächige und im Abstand zueinander liegende Abstandshalter in der Form der Noppen 20 erreicht. Drei im unteren Bereich der Umfangsfläche des zylindrischen Schaftunterteils 2 versetzte Abstandshalter als Noppenreihen 17, 18, 19 sind für eine Zentrierung im zylindrisch vorbereiteten Aufnahmeraum ausreichend. Die weitere Zentrierung und Abstandshalterung im oberen Bereich des Aufnahmeraums wird über die an Corticalis medial des Schenkelhalsstumpfes anliegende Noppenreihe 21 erhalten. Um mögliche Durchmessertoleranzen nach der zylindrischen Vorbereitung des Aufnahmeraums auszugleichen und für eine sichere Zentrierung bezüglich der Zylindermittenachse sind Noppenreihen 17, 18, 19 mit einer gewissen, insgesamt geringen Längserstreckung verwendet. Durch deren gegenseitigen Höhenversatz und die Zwischenräume 23 zwischen den Noppen 20 wird die Zementschicht nur wenig unterbrochen.

Die eingesetzte Schaftoberfläche ist zudem insgesamt rauh gestaltet mit einer bevorzugten Porentiefe von 0,2 bis 0,6 mm. Ebenso ist der zylindrische Aufnahmeraum an seiner Innenseite mit Rillen in Form von Spiralrillen oder Längsrillen versehen, um eine verbesserte Zementverzahnung zwischen Aufnahmeraum bzw. Markraum und Schaftoberfläche zu erhalten.

Die gleichmäßige Zementschichtdicke von ca. 2,5 mm, die geringen Unterbrechungen der Zementschicht an nur wenigen Stellen und die verbesserte Zementverzahnung durch rauhe Oberflächen führen zu einer hohen primären und Langzeitstabilität.

## Patentansprüche

1. Hüftkopf-Schaft-Prothese (1),
mit einem länglichen Schaftteil, das im Markraum eines Oberschenkelhalsstumpfes implantierbar und durch eine das Schaftteil (2,3) umgebende Zementschicht im Markraum fixierbar ist,
mit einem am Schaftteil (2,3) anschließenden, gegenüber der Längsachse (12), des Schaftteils seitlich abstehenden Hüftkopfteil (9) und
mit an der Umfangsfläche des Schaftteils (2, 3) verteilten, im Vergleich zur Größe der Umfangsfläche kleinflächigen Erhebungen, wobei das Schaftoberteil (3) an der lateralseite als gerade Verlängerung (4) des zylindrischen Schaftunterteils (2) ausgebildet ist und im Bereich des Schaftoberteils (3) die Medialseite konisch mit einem konkaven Bogen (5) erweitert ist,
dadurch gekennzeichnet,
daß das Schaftunterteil (2) zylindrisch geformt ist, wobei ausschließlich im distalen Endbereich des Schaftunterteils (2) am Umfang verteilt Erhebungen als untere Abstandshalter (17, 18, 19) angebracht sind für eine Zentrierung in einem zylindrisch im Durchmesser des Außendurchmessers des Schaftunterteils (2) zuzüglich der abstehenden Abstandshalter (17, 18, 19) aufgebohrten Markraum des Oberschenkelhalsstumpfes als Aufnahmeraum, und daß ausschließlich medial an konkaven Bogen (5) zusätzlich wenigstens eine Erhebung als oberer Abstandshalter (21) zur Anlage an die Corticalis im Markraum angebracht ist, so daß aufgrund der Abstandshalteranordnung (17, 18, 19, 21) ein gleichmäßiger Spalt und damit eine gleichmäßige Zementschichtdicke mit nur geringen Unterbrechungen zwischen Aufnahmeraum und Schaft für eine hohe primäre und Langzeitstabilität hergestellt ist.

2. Hüftkopf-Schaft-Prothese nach Anspruch 1, dadurch gekennzeichnet, daß die Erhebungen in der Form von in Längsrichtung des Schaftteils verlaufenden, schmalen, im Vergleich zur Schaftlänge jedoch relativ kurzen Abstandshaltern (17, 18, 19, 21) ausgebildet sind.

3. Hüftkopf-Schaft-Prothese nach Anspruch 1 oder 2, dadurch gekennzeichnet, daß drei am Umfang des Schaftunterteils (2) verteilte Abstandshalter vorgesehen sind.

4. Hüftkopf-Schaft-Prothese nach einem der Ansprüche 1 bis 3, dadurch gekennzeichnet, daß jeder Abstandshalter als Noppenreihe (17, 18, 19, 21) ausgebildet ist, wobei zwischen den Noppen (20) Zwischenräume (23) liegen.

5. Hüftkopf-Schaft-Prothese nach einem der Ansprüche 1 bis 4, dadurch gekennzeichnet, daß die Abstandshalter bzw. die Noppenreihen (17, 18, 19) gegeneinander in der Schaft-Längsrichtung (12) bzw. in ihrer Höhe versetzt angebracht sind, um eine möglichst geringe Umfangsunterbrechung der Zementschicht zu erhalten.

6. Hüftkopf-Schaft-Prothese nach einem der Ansprüche 1 bis 5, dadurch gekennzeichnet, daß die Höhe der Abstandshalter bzw. Noppen (20) im Bereich zwischen einer erforderlichen Mindestdicke einer Zementschicht von ca. 1,5 mm und 3,5 mm, bevorzugt bei 2,5 mm, liegt.

7. Hüftkopf-Schaft-Prothese nach einem der Ansprüche 1 bis 6, dadurch gekennzeichnet, daß die Schaftoberfläche insgesamt rauh gestaltet ist mit einer bevorzugten Porentiefe von 0,2 bis 0,6 mm.

8. Hüftkopf-Schaft-Prothese nach einem der Ansprüche 1 bis 7, dadurch gekennzeichnet, daß sich die gegenüberliegende Medialseite mit einem konkaven Bogen (5), bevorzugt mit einem Tangentenneigungswinkel (8) von 15° gegenüber der Längsachse (12), erweitert, so daß dadurch etwa eine Dreiecksfläche in der Dicke des Durchmessers des zylindrischen Schaftunterteils (2) mit einer senkrechten Begrenzung (4), einer waagrechten Begrenzung (6) und einer bogenförmigen Begrenzung (5) aufgespannt ist, wobei sich das Hüftkopfteil bevorzugt als Konus (10) mit einer gegenüber der Längsachse (12) um 40° geneigten Konusachse (11), an der Stelle des Zusammentreffens der waagrechten (6) und bogenförmigen Begrenzung (5) anschließt.

9. Hüftkopf-Schaft-Prothese nach einem der Ansprüche 1 bis 8, dadurch gekennzeichnet, daß unterhalb des Hüftkopfteils (10) ein Kragen (15), bevorzugt mit einer Neigung (16) von 50° gegenüber der Längsachse (12), zur Auflage an der Resektionsebene des Schenkelhalses angeformt ist.

## Claims

1. Femoral head/shaft prosthesis (1), having an elongate shaft part which can be implanted in the marrow space of a femoral neck stump and can be fixed in the marrow space by a layer of cement surrounding the shaft part (2, 3), having a femoral head part (9) which adjoins the shaft part (2, 3) and projects laterally relative to the longitudinal axis (12) of the shaft part, and having elevations which are distributed over the circumferential surface of the shaft part (2, 3) and have a small surface area in comparison to the size of the circumferential surface, the upper part (3) of the shaft being constructed on the lateral side as a straight continuation (4) of the cylindrical lower part (2) of the shaft, and the medial side being widened conically with a concave curve (5) in the region of the upper part (3) of the shaft, characterised in that the lower part (2) of the shaft is of cylindrical shape, elevations distributed over the circumference being applied solely in the distal end region of the lower part (2) of the shaft as lower spacers (17, 18, 19) for centring in a marrow space of the femoral neck stump as a receiving space, said marrow space having been bored open cylindrically with the diameter of the outside diameter of the lower part (2) of the shaft and the projecting spacers (17, 18, 19) in addition, and in that additionally at least one elevation is applied solely medially on the concave curve (5) as an upper spacer (21) for resting against the cortex layer in the marrow space so that, due to the spacer arrangement (17, 18, 19, 21), an even gap and thus an even thickness of the layer of cement are produced with only slight interruptions between the receiving space and the shaft for high primary and long-term stability.

2. Femoral head/shaft prosthesis according to Claim 1, characterised in that the elevations are constructed in the form of spacers (17, 18, 19, 21) which run in the longitudinal direction of the shaft part and are narrow, but relatively short in comparison to the shaft length.

3. Femoral head/shaft prosthesis according to Claim 1 or 2, characterised in that three spacers are provided which are distributed over the circumference of the lower part (2) of the shaft.

4. Femoral head/shaft prosthesis according to one of Claims 1 to 3, characterised in that each spacer is constructed as a row of knobs (17, 18, 19, 21), gaps (23) lying between the knobs (20).

5. Femoral head/shaft prosthesis according to one of Claims 1 to 4, characterised in that the spacers or the rows of knobs (17, 18, 19) are applied so as to be offset relative to one another in the longitudinal direction (12) of the shaft or in their height in order to obtain as little interruption in the circumference of the layer of cement as possible.

6. Femoral head/shaft prosthesis according to one of Claims 1 to 5, characterised in that the height of the spacers or knobs (20) lies in the range between a required minimum thickness of a layer of cement of about 1.5 mm and 3.5 mm, preferably 2.5 mm.

7. Femoral head/shaft prosthesis according to one of Claims 1 to 6, characterised in that the shaft surface is designed in total to be rough with a preferred pore depth of 0.2 to 0.6 mm.

8. Femoral head/shaft prosthesis according to one of Claims 1 to 7, characterised in that the opposite medial side is widened with a concave curve (5), preferably at a tangential angle of inclination (8) of 15° relative to the longitudinal axis (12), so that, as a result, an approximately triangular area is defined with the thickness of the diameter of the cylindrical lower part (2) of the shaft with a vertical boundary (4), a horizontal boundary (6) and a curved boundary (5), the femoral head part, preferably as a cone (10) with a conical axis (11) which is inclined by 40° relative to the longitudinal axis (12), adjoining the point of intersection of the horizontal (6) and curved boundary (5).

9. Femoral head/shaft prosthesis according to one of Claims 1 to 8, characterised in that a collar (15) is moulded onto the resection plane of the femoral neck below the femoral head part (10), preferably at an inclination (16) of 50° relative to the longitudinal axis (12).

## Revendications

1. Prothèse de hanche à tête et à tige (1), comportant:
une partie tige de forme allongée qui peut être implantée dans la cavité médullaire d'un moignon de col de fémur et qui peut être fixée dans la cavité médullaire par une couche de ciment entourant la partie tige (2, 3),
une partie tête coxale (9) qui fait suite à la partie tige (2, 3) et qui s'écarte latéralement par rapport à l'axe longitudinal (12) de la partie tige, et
des saillies qui sont réparties sur la surface circonférentielle de la partie tige (2, 3) et qui ont une petite surface par rapport à la dimension de la surface circonférentielle,
la partie supérieure (3) de la tige étant réalisée, du côté externe, sous forme de prolongement en ligne droite (4) de la partie inférieure cylindrique (2) de la tige, son côté interne s'élargissant, au niveau de la partie supérieure (3) de la tige, en forme de cône avec une courbure concave (5),
caractérisée en ce que la partie inférieure (2) de la tige a une forme cylindrique, en ce que des saillies, réparties sur la circonférence, exclusivement dans la région d'extrémité distale de la partie inférieure (2) de la tige, sont disposées en tant qu'éléments d'écartement inférieurs (17, 18, 19) pour un centrage dans la cavité médullaire du moignon du col de fémur, servant de cavité de logement et réalisée de sorte que son diamètre corresponde au diamètre extérieur de la partie inférieure (2) de la tige plus les éléments d'écartement saillants (17, 18, 19), et en ce qu'il est en plus disposé, exclusivement du côté interne au niveau de la courbure concave (5), au moins une saillie servant d'élément d'écartement supérieur (21) destiné à s'appliquer sur la corticale dans la cavité médullaire, de sorte qu'il soit créé entre la cavité de logement et la tige, en conséquence de la disposition des éléments d'écartement (17, 18, 19, 21), une fente régulière et, par suite, une épaisseur régulière de la couche de ciment ne présentant que de petites interruptions, en vue d'une stabilité élevée, initialement et à long terme.

2. Prothèse de hanche à tête et à tige selon la revendication 1, caractérisée en ce que les saillies sont réalisées sous la forme d'éléments d'écartement (17, 18, 19, 21) s'étendant dans la direction longitudinale de la partie tige, minces mais relativement courts en comparaison de la longueur de la tige.

3. Prothèse de hanche à tête et à tige selon la revendication 1 ou 2, caractérisée en ce qu'il est prévu trois éléments d'écartement répartis sur la circonférence de la partie inférieure (2) de la tige.

4. Prothèse de hanche à tête et à tige selon l'une quelconque des revendications 1 à 3, caractérisée en ce que chaque élément d'écartement est réalisé sous forme de rangée de plots (17, 18, 19, 21, des intervalles (23) étant ménagés entre les plots (20).

5. Prothèse de hanches à tête et à tige selon l'une quelconque des revendications 1 à 4, caractérisée en ce que les éléments d'écartement ou les rangées de plots (17, 18, 19) sont disposés avec un décalage en hauteur, c'est-à-dire dans la direction longitudinale (12) de la tige, afin de maintenir une interruption aussi petite que possible de la couche de ciment dans la direction circonférentielle.

6. Prothèse de hanche à tête et à tige selon l'une quelconque des revendications 1 à 5, caractérisée en ce que la hauteur des éléments d'écartement ou plots (20) se situe dans la gamme de l'épaisseur minimale nécessaire d'une couche de ciment, c'est-à-dire entre 1,5 et 3,5 mm environ, de préférence de l'ordre de 2,5 mm.

7. Prothèse de hanche à tête et à tige selon l'une quelconque des revendications 1 à 6, caractérisée en ce que la surface complète de la tige est rendue rugueuse, avec une profondeur préférée des pores de 0,2 à 0,6 mm.

8. Prothèse de hanche à tête et à tige selon l'une quelconque des revendications 1 à 7, caractérisée en ce que son côté interne opposé s'élargit avec une courbure concave (5) dont la tangente forme de préférence un angle d'inclinaison (8) de 15° avec l'axe longitudinal (12), d'où il résulte un plat à peu près triangulaire ayant une épaisseur égale au diamètre de la partie inférieure cylindrique (2) de la tige et présentant une limite verticale (4), une limite horizontale (6) et une limite courbe (5), la partie tête coxale, réalisée de préférence sous forme de cône (10) dont l'axe (11) est incliné de 40° par rapport à l'axe longitudinal (12), se raccordant au point de rencontre de la limite horizontale (6) et de la limite courbe (5).

9. Prothèse de hanche à tête et à tige selon l'une quelconque des revendications 1 à 8, caractérisée en ce qu'une collerette (15), destinée à prendre appui sur le plan de résection du col de fémur, est formée au-dessous de la partie tête coxale (10), de préférence avec une inclinaison (16) de 50° par rapport à l'axe longitudinal (12).
